# FASCICULE DE BREVET EUROPEEN

(11) **EP 1 372 772 B1**
(45) Date de publication et mention de la délivrance du brevet: **25.05.2005**
(21) Numéro de dépôt: 02714276.9
(22) Date de dépôt: 05.03.2002
(51) Int. Cl.: A61M 5/32

(54) **SERINGUE A SECURITE RENFORCEE**
SICHERHEITSSPRITZE
SYRINGE WITH IMPROVED SAFETY

(30) Priorité: 05.03.2001 FR 0102985; 05.03.2001 FR 0102983
(43) Date de publication de la demande: 02.01.2004
(73) Titulaire: Becton Dickinson France, 38800 Le Pont-de-Claix (FR)
(72) Inventeur: BARRELLE, Laurent, F-38250 SAINT NIZIER DU MOUCHEROTTE (FR); JANSEN, Hubert, F-38560 HAUTE JARRIE (FR)
(74) Mandataire: Brédeville, Odile Marie
(86) Numéro de dépôt international: PCT/FR2002/000789
(87) Numéro de publication internationale: WO 2002/070053

(56) Documents cités:
- EP-A- 1 090 652
- WO-A-00/56383
- US-A- 5 242 416
- US-A- 5 383 857

## Description

La présente invention concerne un dispositif à usage médical, notamment une seringue, à sécurité renforcée.

L'invention est susceptible d'être appliquée à tout dispositif comprenant :
(i) un organe acéré ou tranchant, notamment une aiguille de seringue, destiné à être introduit dans un tissu du patient ou à être mis en contact avec un fluide corporel du patient, et donc à être éventuellement contaminé par ce tissu ou ce fluide corporel ;
(ii) un étui de protection, engagé autour d'une pièce du dispositif, notamment un corps de seringue, comprenant ledit organe acéré ou tranchant ; cet étui et cette pièce sont mobiles l'un par rapport à l'autre entre une position d'utilisation du dispositif, dans laquelle ledit organe acéré ou tranchant est exposé, et une position de sécurité, dans laquelle cet organe est entouré par l'étui, de manière à prévenir tout risque de piqûre ou de coupure après utilisation, et donc de contamination éventuelle, de l'utilisateur par cet organe ; et
(iii) des moyens de déplacement permettant, notamment par action manuelle, de déplacer ladite pièce et ledit étui l'un par rapport à l'autre entre lesdites positions d'utilisation et de sécurité.

L'invention est en priorité destinée à être appliquée à une seringue, l'organe acéré ou tranchant étant l'aiguille de cette seringue.

Le document EP 1 090 652 révèle un dispositif conforme au préambule de la revendication 1.

Le dispositif selon ce document a toutefois pour inconvénient de ne pas assurer une parfaite sécurité contre la réutilisation de la seringue.

Le document WO 00/56383 révèle un dispositif dans lequel rien n'empêche, en fin d'injection, un déplacement de l'étui par rapport au corps de seringue. Ce déplacement, s'il est réalisé, a non seulement pour effet de découvrir à nouveau l'aiguille, mais également, par le recul du piston, de permettre un nouveau remplissage de la seringue. Le dispositif selon ce document a donc également pour inconvénient de ne pas assurer une parfaite sécurité contre la réutilisation de la seringue.

Les dispositifs existants présentent également différents inconvénients liés soit à leur relative complexité de structure, soit à des problèmes de fabrication résultant des tolérances dimensionnelles de leurs pièces constitutives ou des tolérances d'assemblage de ces pièces, soit à leur absence d'adaptabilité à des corps de seringue de différents diamètres, soit à l'existence de jeux entre lesdites pièces constitutives.

La présente invention vise à remédier à l'ensemble de ces inconvénients.

Son objectif principal est donc de fournir un dispositif tel que précité, susceptible de procurer à l'utilisateur une sécurité parfaite, ou tout au moins renforcée, contre le risque de piqûre ou de coupure accidentelle après utilisation, en éliminant toute nécessité d'action distincte et séparée pour le déplacement de l'étui de protection en position de sécurité, et en éliminant toute possibilité de contrôle de ce déplacement.

Un autre objectif de l'invention est de parvenir à l'objectif indiqué ci-dessus sans complexifier outre mesure la structure ou l'assemblage du dispositif, voire même en simplifiant cette structure et cet assemblage par rapport aux dispositifs connus.

Le dispositif concerné comprend, de manière connue en soi :
(i) un organe acéré ou tranchant, notamment une aiguille, destiné à être introduit dans un tissu du patient ou à être mis en contact avec un fluide corporel du patient, et donc à être éventuellement contaminé par ce tissu ou ce fluide corporel ;
(ii) un étui de protection, engagé autour d'une pièce du dispositif, notamment un corps de seringue, comprenant ledit organe acéré ou tranchant ; cet étui et cette pièce sont mobiles l'un par rapport à l'autre entre une position d'utilisation du dispositif, dans laquelle ledit organe acéré ou tranchant est exposé, et une position de sécurité, dans laquelle cet organe est entouré par l'étui, de manière à prévenir tout risque de piqûre ou de coupure après utilisation, et donc de contamination éventuelle, de l'utilisateur par cet organe ; et
(iii) des moyens de déplacement permettant, notamment par action manuelle, de déplacer ladite pièce et ledit étui l'un par rapport à l'autre entre lesdites positions d'utilisation et de sécurité ;
   - l'étui de protection comprenant au moins une lumière ou saignée longitudinale ;
   - la pièce comportant l'organe acéré ou tranchant étant reliée à une première pièce de prise d'appui pouvant coulisser par rapport à l'étui de protection, cette première pièce de prise d'appui présentant au moins une partie de prise d'appui, engagée au travers de ladite lumière ou saignée et faisant saillie au-delà de l'étui de protection, qui sert à la prise d'appui de deux doigts, notamment l'index et le majeur, de la main de l'utilisateur ;
   - le dispositif comprenant une deuxième pièce de prise d'appui, notamment une tige de piston, présentant une partie de prise d'appui pour un troisième doigt, notamment le pouce, de l'utilisateur, et
   - le dispositif comprenant des moyens ruptibles, propres à se rompre au-delà d'un seuil de forces antagonistes exercées sur lesdites parties de prise d'appui respectives desdites première et deuxième pièces de prise d'appui, la rupture de ces moyens générant un mouvement relatif brusque de ladite pièce comportant l'organe acéré ou tranchant et dudit étui de protection, tel que cette pièce et cet étui sont amenés en position de sécurité à l'issue de ce mouvement brusque.

Selon l'invention, le dispositif comprend en outre des moyens de verrouillage de la pièce comportant l'organe acéré ou tranchant et de l'étui de protection en position de sécurité.

L'action permettant d'amener ladite pièce comportant l'organe acéré ou tranchant et l'étui de protection en position de sécurité est ainsi réalisée par la même prise manuelle que la prise par laquelle le dispositif est maintenu au cours de son utilisation, et peut donc être réalisée dans le prolongement de cette utilisation.

Ledit mouvement brusque limite ou même interdit tout contrôle intempestif du déplacement relatif de la pièce comportant l'organe acéré ou tranchant et de l'étui.

Le verrouillage de la pièce comportant l'organe acéré ou tranchant et de l'étui dans la position de sécurité est assuré, ou tout au moins le déverrouillage de cette pièce et de cet étui est rendu virtuellement impossible.

Selon une forme de réalisation préférée de l'invention, le dispositif étant une seringue,
- ladite pièce comportant l'organe acéré ou tranchant est formée par un corps de seringue, présentant une aiguille sur son extrémité distale et un moyen d'assemblage, tel qu'une collerette, sur son extrémité proximale ;
- ladite première pièce de prise d'appui est formée par une bague comprenant une partie logée à l'intérieur de l'étui de protection, munie de moyens d'assemblage complémentaires de celui du corps de seringue pour assurer une liaison axiale entre cette bague et ce corps de seringue, et une partie dépassant de l'étui, comprenant deux saillies en forme d'ailettes, par exemple diamétralement opposées, formant lesdites parties de prise d'appui destinées à recevoir respectivement l'index et le majeur de l'utilisateur ;
- ladite deuxième pièce de prise d'appui est formée par la tige du piston de la seringue ; cette tige de piston comprend deux parties télescopiques, dont une, distale, est reliée au piston de la seringue, et dont l'autre, proximale, comporte une tête, formant ladite partie de prise d'appui du pouce de l'utilisateur ; et
- lesdits moyens ruptibles sont constitués par au moins un pont de matière reliant les deux parties télescopiques de la tige de piston, ledit seuil de force de rupture de ce ou ces ponts étant supérieur à la force nécessaire au déplacement du piston mais inférieur aux forces antagonistes qu'un utilisateur est susceptible d'exercer manuellement sur lesdites saillies et ladite tête.

Le piston peut ainsi être actionné pour réaliser l'injection, puis, lorsque ce piston est en butée contre le fond du corps de la seringue ou lorsque la tête du piston vient en butée contre l'étui, lesdites forces antagonistes peuvent être exercées au-delà dudit seuil de forces pour rompre le ou lesdits ponts et provoquer ainsi le déplacement relatif du corps de seringue et de l'étui de protection vers ladite position de sécurité.

De préférence, la tige du piston présente une longueur telle que la tête de piston n'est pas en butée contre le bord proximal de l'étui lorsque le piston est en butée contre le fond du corps de seringue.

Les moyens de verrouillage sont de préférence du type à enclenchement, notamment à encliquetage, et sont positionnés de sorte que le verrouillage se produit automatiquement au moment où la pièce comportant l'organe acéré ou tranchant et l'étui de protection atteignent ladite position de sécurité.

Avantageusement,
- la bague présente une paroi périphérique définissant un logement propre à recevoir étroitement, en fin d'injection, la tête que comprend la tige d'actionnement du piston de la seringue ;
- des moyens de verrouillage sont aménagés dans ledit logement ;et
- la tête de la tige de piston comprend des moyens de verrouillage propres à venir en prise avec les moyens de verrouillage de la bague lorsque cette tête est reçue dans ledit logement.

La tête de la tige de piston est ainsi engagée et verrouillée dans ledit logement en fin d'injection, de sorte qu'il existe non seulement une immobilisation de la tige de piston dans cette position de fin d'injection mais également qu'il n'existe aucune prise sur la tête de piston permettant de séparer lesdits moyens de verrouillage.

Le bord proximal de ladite paroi périphérique présente avantageusement un chanfrein pour faciliter l'engagement de ladite tête dans ledit logement.

Selon une forme de réalisation préférée de l'invention, les moyens de verrouillage que comprend la paroi périphérique sont constitués par au moins une nervure aménagée dans cette paroi, et les moyens de verrouillage que comprend ladite tête sont constitués par un rebord correspondant faisant saillie radialement de cette tête vers l'extérieur, propre à être engagé et verrouillé par encliquetage derrière cette ou ces nervures.

De préférence,
- ladite partie de la bague située à l'intérieur de l'étui comprend deux parties de maintien, par exemple diamétralement opposées, en forme de fourche c'est-à-dire comprenant chacune une paire de bras et un corps de liaison reliant ces bras à la bague, les bras de chaque fourche étant conformés de manière à pouvoir entourer partiellement le corps de la seringue ; et
- lesdits bras sont réalisés en un matériau présentant un degré de souplesse élastique tel qu'ils peuvent être déformés élastiquement de manière à pouvoir recevoir entre eux, et enserrer, des corps de seringue de diamètres extérieurs différents.

Le dispositif selon l'invention peut ainsi comprendre des pièces standardisées et recevoir des corps de seringue de différents diamètres extérieurs, en fonction des besoins.

Chacun des bras d'une partie de maintien peut comprendre au moins un bossage faisant saillie radialement vers l'intérieur, pour assurer l'immobilisation, et éventuellement le centrage, d'un corps de seringue d'un diamètre réduit.

Un chanfrein peut être aménagé dans lesdits bras du côté de la bague par lequel un corps de seringue est destiné à être introduit au travers de cette bague.

Ce chanfrein permet de faciliter l'engagement d'un corps de seringue entre lesdits bras.

Au moins une desdites parties de maintien peut présenter une structure flexible, notamment au niveau de son corps de liaison, de manière à faciliter l'assemblage du corps de seringue à la bague, en particulier la réception de la collerette proximale que comprend ce corps de seringue pour son montage dans la seringue.

Lesdites parties de maintien peuvent être conformées de manière à plaquer les moyens d'assemblage du corps de seringue contre la bague ou une portion déterminée de celle-ci. En particulier lesdits bras peuvent être inclinés vers la bague.

Avantageusement, l'étui de protection présente des moyens de calage permettant de caler latéralement un corps de seringue mis en place dans cet étui, ces moyens de calage étant mobiles radialement de manière à pouvoir caler des corps de seringue de diamètres extérieurs différents.

Ces moyens de calage peuvent en particulier être aménagés du côté distal de l'étui de protection, pour caler latéralement la partie distale d'un corps de seringue.

Selon une forme de réalisation préférée de l'invention dans ce cas, l'étui de protection présente au moins deux fentes en forme de "U" diamétralement opposées, qui individualisent chacune une patte mobile radialement, la face interne de chacune de ces pattes présentant, du côté de l'extrémité de la patte non reliée à l'étui de protection, un bossage faisant saillie à l'intérieur de cet étui.

Le corps de seringue vient en appui contre ces bossages lors de sa mise en place dans l'étui de protection. Dans le cas de corps de seringue de plus grands diamètres extérieurs susceptibles d'être utilisés avec la seringue selon l'invention, les pattes se déforment radialement vers l'extérieur de manière à permettre l'effacement adéquat desdits bossages.

Avantageusement, le corps de liaison que comprend chaque partie de maintien est réalisé en un matériau présentant un degré de souplesse élastique, de telle sorte que ce corps de liaison présente une souplesse dans le sens radial de la bague, cette souplesse étant telle qu'elle permet de placer les bras de la partie de maintien selon plusieurs positions radiales distinctes.

Cette souplesse contribue ainsi à élargir la gamme de diamètres de corps de seringue pouvant être reçus par la bague, et à assurer un parfait maintien et un parfait centrage d'un corps de seringue d'un diamètre déterminé.

Selon une forme de réalisation préférée de l'invention dans ce cas, chaque corps de liaison présente une forme coudée, c'est-à-dire comprend une partie reliée auxdits bras, disposée sensiblement perpendiculairement à l'axe de la bague, et une partie reliée à la bague, disposée sensiblement parallèlement à l'axe de la bague.

Avantageusement,
- le corps de liaison de l'une des parties de maintien est reliée à la bague à proximité de l'une des saillies précitées de prise d'appui d'un ou des doigts de l'utilisateur et le corps de liaison de l'autre partie de maintien est relié à la bague à proximité de l'autre de ces saillies, et
- au moins une partie de ces corps de liaison est conformée de manière à faire saillie au-delà de la paroi de l'étui de protection.

La présence de ces corps de liaison permet ainsi d'écarter les doigts de l'utilisateur par rapport à la paroi de l'étui de protection, afin d'empêcher que ces doigts viennent frotter contre l'étui de protection lors du mouvement brusque précité, et qu'ils viennent ainsi contrarier ce mouvement.

De préférence,
- le corps de seringue présente une collerette proximale pour son assemblage à la bague ;
- la partie de la bague située à l'intérieur de l'étui comprend les parties de maintien précitées, et
- la bague comprend deux parties d'assemblage de forme courbe, disposées de manière à former substantiellement un anneau ; ces parties d'assemblage sont reliées à la bague au niveau de zones périphériques et sont réalisées en un matériau présentant un degré de souplesse élastique ; les zones périphériques de ces parties d'assemblage sont situées l'une par rapport à l'autre à une distance supérieure au diamètre externe de ladite collerette tandis que les zones médianes de ces parties d'assemblage sont situées l'une par rapport à l'autre, à l'état non déformé de ces parties d'assemblage, à une distance inférieure au diamètre extérieur de la collerette ; ces zones médianes présentent, du côté de la bague par lequel le corps de seringue est engagé au travers des parties d'assemblage, des chanfreins conformés pour permettre l'effacement radial de ces zones médianes au moment de cet engagement.

L'engagement de la collerette entre ces parties d'assemblage provoque la déformation élastique de celles-ci au niveau desdites zones médianes, dont les chanfreins permettent l'effacement radial. Une fois la collerette engagée au-delà de ces parties d'assemblage, le rappel élastique du matériau constituant ces parties d'assemblage réalise un verrouillage du corps de seringue.

L'invention se rapporte à un dispositif à usage médical, avec sécurité, comprenant
(a) un sous-ensemble du type seringue, comprenant un corps allongé selon un axe de référence ; une aiguille disposée axialement, communiquant avec l'intérieur dudit corps, à l'extrémité distale de ce dernier ; un piston déplaçable à l'intérieur dudit corps jusqu'à une position distale en butée ; et une tige de piston émergeant à l'extrémité proximale dudit corps, avec une partie proximale de prise d'appui ;
(b) un sous-ensemble de sécurité, comprenant un étui de protection monté autour du corps, déplaçable axialement par rapport à ce dernier entre deux positions, à savoir une position d'exposition dans laquelle l'aiguille est exposée et une position de sécurité dans laquelle l'aiguille est entièrement contenue à l'intérieur dudit étui de protection ;
selon l'invention, en combinaison :
(i) l'étui de protection est prolongé par une portion proximale au-delà de l'extrémité proximale du corps dans la position d'exposition dudit étui de protection, la tige de piston émergeant de l'extrémité proximale de ladite portion proximale ;
(ii) une bague d'actionnement s'étend radialement à partir de l'extrémité proximale du corps, en étant solidaire de cette dernière, et comporte une partie externe de prise d'appui, de part et d'autre du corps ;
(iii) des moyens de coulissement axial entre la bague d'actionnement et la portion proximale de l'étui de protection, ladite portion proximale étant montée librement en translation au travers de la bague ;
(iv) des moyens de rétraction axiale de la tige de piston sur elle-même, par poussée axiale sur la partie proximale de prise d'appui lorsque le piston est dans sa position distale en butée, en sorte que ladite partie proximale de prise d'appui vient en butée contre l'extrémité proximale de l'étui de protection, puis déplace ce dernier vers sa position de sécurité, toujours par poussée.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 en est une vue en perspective, sous forme d'une seringue, avant injection du produit que contient cette seringue ;
la figure 2 en est une vue de côté, en coupe longitudinale ;
la figure 3 est une vue à échelle agrandie, d'une portion de la tige de piston que comprend la seringue ;
la figure 4 est une vue de détail d'un étui de protection que comprend la seringue, à échelle agrandie et en coupe longitudinale ;
la figure 5 est une vue de côté, à échelle agrandie, de cet étui et d'une bague d'actionnement que comprend la seringue ;
la figure 6 est une vue axiale de cet étui et de cette bague, du côté proximal de la seringue ;
la figure 7 est une vue en perspective de cette même bague ;
la figure 8 est une vue axiale de cette bague, et d'un corps de seringue assemblé à elle, du côté distal de la seringue ;
la figure 9 est une vue similaire à la figure 8, après mise en place dans la bague d'un corps de seringue de plus gros diamètre que le corps de seringue montré sur la figure 8 ;
la figure 10 est une vue de la seringue similaire à la figure 2, une fois l'injection réalisée ; et
la figure 11 est une vue de côté, à échelle agrandie, de la partie proximale de la seringue, une fois l'injection réalisée.

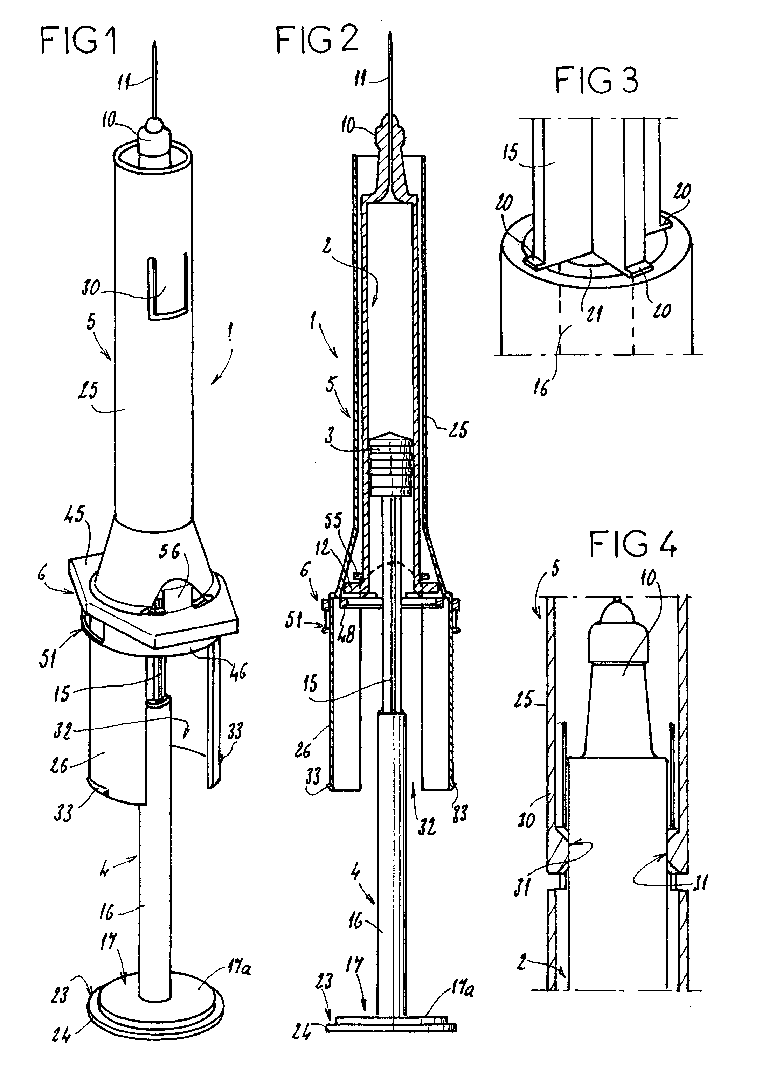

Les figures 1 et 2 représentent une seringue 1 à sécurité renforcée, comprenant un corps de seringue 2, un piston 3, une tige de piston 4, un étui de protection 5 et une bague 6 d'actionnement de l'étui 5.

Le corps de seringue 2 est de type classique, réalisé par exemple en verre. Il comprend un embout distal 10 comportant l'aiguille d'injection 11 et une collerette proximale 12, faisant saillie radialement vers l'extérieur, qui permet l'assemblage du corps 2 avec la bague 6, comme cela sera décrit plus loin. Le montage de l'aiguille 11 peut être réalisé autrement qu'au moyen d'un tel embout 10.

Le piston 3 est de type classique.

La tige de piston 4 comprend deux parties télescopiques 15, 16, dont celle distale 15 présente une section transversale en forme de croix et dont celle proximale 16 présente une forme tubulaire, propre à recevoir la partie 15 à coulissement. La partie 15 peut avoir une section d'une autre forme qu'en croix, ou la partie 15 peut présenter une forme tubulaire et la partie 16 présenter une section transversale en forme de croix.

La partie 16 comprend une tête d'appui 17 pour le pouce de l'utilisateur.

Comme le montre plus particulièrement la figure 3, chacune des quatre ailes que présente la partie 15 est reliée à la partie 16 par au moins un pont de matière 20, de sorte que ces parties 15 et 16 sont normalement maintenues dans une position d'extension, montrée sur les figures 1 et 2.

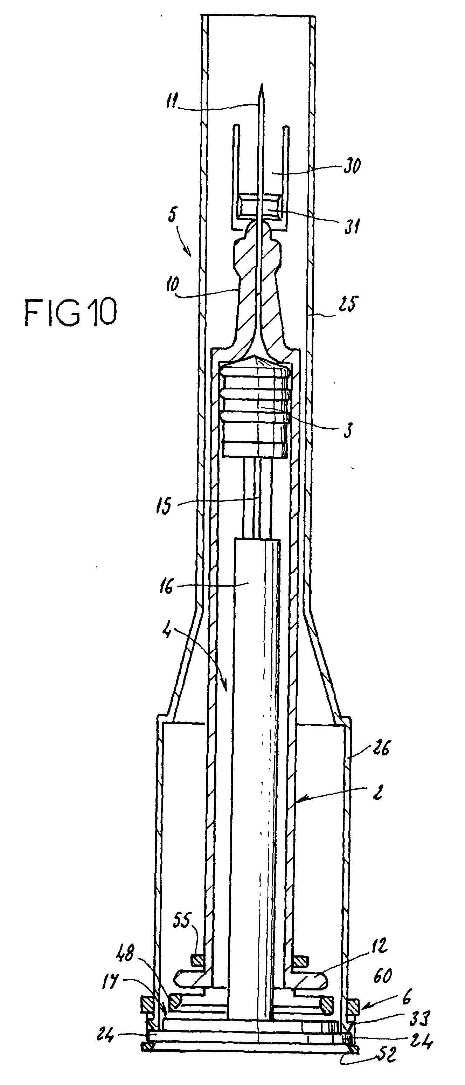

Ces ponts 20 sont suffisamment solides pour résister à la force qu'il est nécessaire d'exercer sur la tige 4 pour faire coulisser le piston 3 dans le corps 2, mais sont propres à se rompre au-delà d'un seuil de forces antagonistes susceptibles d'être exercées sur la bague 6 par deux doigts, notamment l'index et le majeur, de l'utilisateur et sur la tête 17 par un troisième doigt, notamment le pouce, de l'utilisateur, selon le geste classique d'actionnement d'une seringue. Une fois ces ponts 20 rompus, la partie 15 peut coulisser dans la partie 16 jusqu'à la position montrée sur la figure 10.

La partie 15 se prolonge, du côté proximal, au-delà des ponts 20, et cette prolongation 21 présente un diamètre extérieur légèrement inférieur au diamètre intérieur de la partie 16. Cette prolongation 21 permet de rigidifier longitudinalement la tige 4 afin d'éviter que cette tige présente, avant rupture des ponts 20, une zone de faiblesse dans le sens transversal.

La tête 17 présente une forme circulaire et comprend un redan périphérique 23, qui délimite une portion cylindrique 17a de la tête 17 et un rebord 24 faisant saillie radialement vers l'extérieur. Comme le montre la figure 11, le diamètre de cette portion cylindrique 17a est tel que cette portion cylindrique 17a peut être engagée avec ajustement dans la portion proximale 26 de l'étui 5 en fin d'injection.

L'étui 5 présente une portion distale 25 de diamètre interne supérieur au diamètre externe du corps 2 et une portion proximale 26 recevant la bague 6 à coulissement. L'ensemble de cet étui 5 est réalisé en une pièce par moulage d'un matériau synthétique présentant un degré de souplesse élastique telle qu'une matière plastique courante.

Comme le montrent les figures 1 et 4, la portion 25 présente deux fentes en forme de "U" diamétralement opposées, qui individualisent chacune une patte 30, cette patte 30 étant mobile radialement par déformation élastique au niveau de sa base. La face interne de chacune de ces pattes présente, du côté de l'extrémité de la patte 30 non reliée à l'étui 5, un bossage 31 faisant saillie à l'intérieur de cet étui.

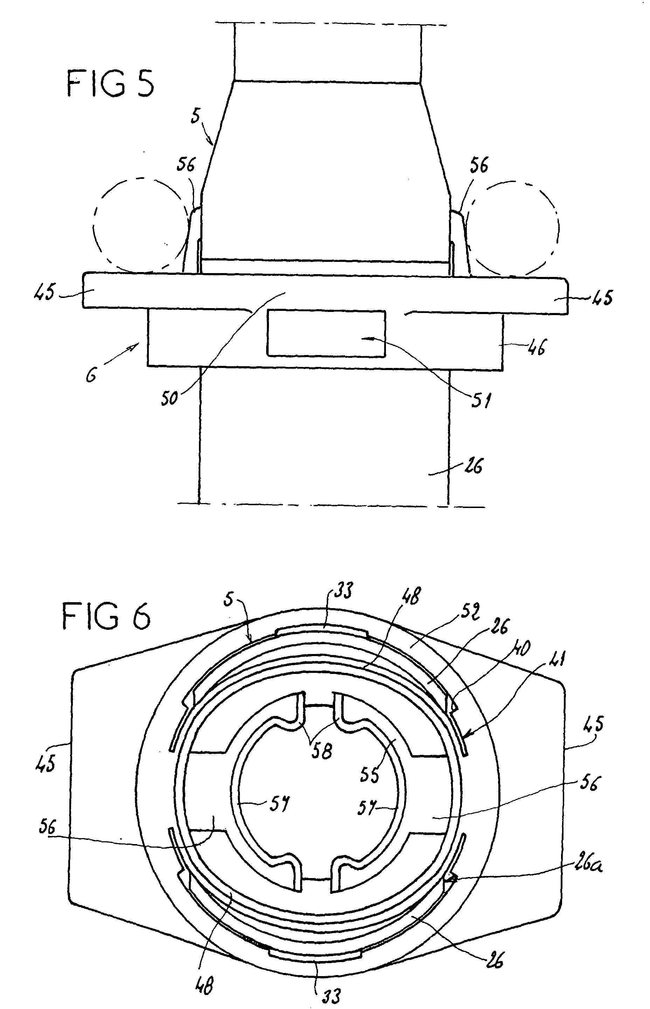

La portion 26 de l'étui présente deux méplats longitudinaux 26a (cf. figure 6) diamétralement opposés, au niveau desquels sont aménagés deux saignées 32 débouchant dans l'extrémité proximale de l'étui 5. Ces saignées 32 sont destinées à recevoir à coulissement des parties 56 de la bague 6, décrites plus loin.

Cette portion 26 présente en outre deux dents d'encliquetage 33 au niveau de son bord d'extrémité, à même d'assurer la rétention de la bague 6 sur l'étui 5, ainsi que cela sera également décrit plus loin.

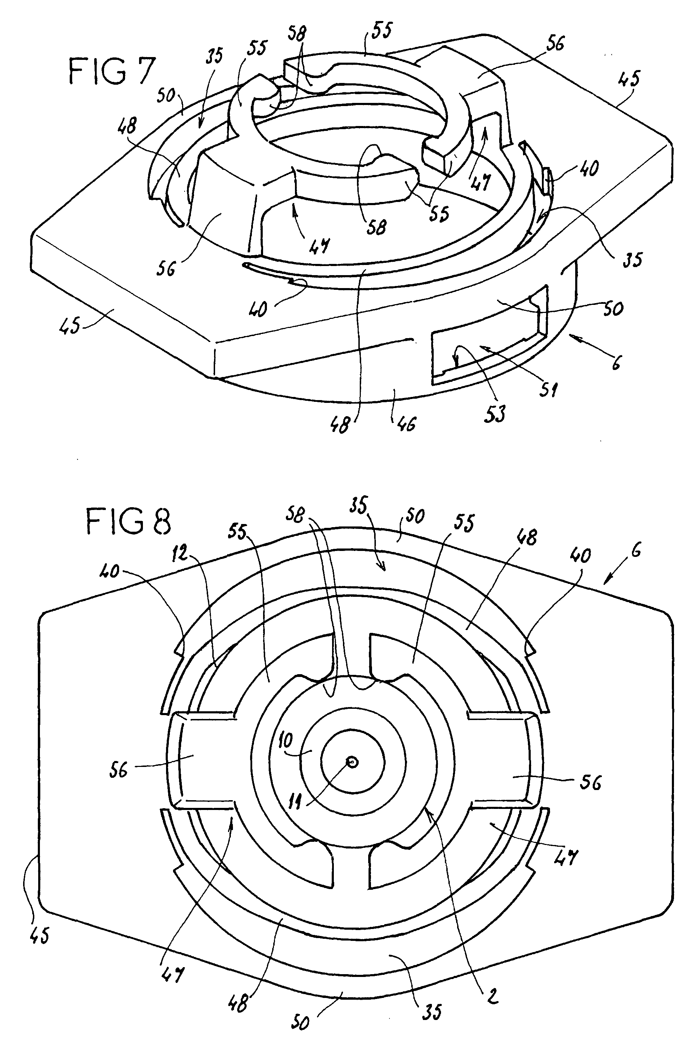

En référence aux figures 6 et 7, il apparaît que la bague 6 comprend deux lumières 35 de forme arquée, disposées symétriquement par rapport à l'axe de cette bague. Comme cela est visible sur la figure 6, ces lumières 35 sont destinées à recevoir les parties de l'étui 5 s'étendant entre lesdites saignées 32.

En retrait des extrémités longitudinales de ces lumières 35, sont formés des épaulements de calage 40, permettant de caler en rotation la bague 6 par rapport à l'étui 5. Chaque lumière 35 se prolonge au-delà de chaque épaulement 40, sous forme d'une fente 41.

La bague 6 présente deux ailettes d'appui 45 diamétralement opposées, faisant saillie radialement vers l'extérieur, une jupe proximale 46, deux parties de maintien 47 et deux parties d'assemblage 48. L'ensemble de cette bague 6 est réalisé en une pièce par moulage d'un matériau synthétique présentant un degré de souplesse élastique telle qu'une matière plastique courante.

Les ailettes 45 sont dimensionnées pour former des surfaces d'appui pour l'index et le majeur de l'utilisateur (schématisés par des cercles en traits interrompus sur la figure 5), et sont raccordées l'une à l'autre par des portions intermédiaires 50 de la bague 6.

La jupe 46 définit un logement propre à recevoir étroitement, en fin d'injection, la tête 17, comme le montrent les figures 10 et 11. Elle présente deux fenêtres 51 diamétralement opposées et un chanfrein proximal 52, visible notamment sur la figure 11.

Comme cela apparaît sur les figures 10 et 11, la bague 6 est destinée à être engagée sur l'extrémité proximale de l'étui 5 jusqu'au-delà des dents 33, ce qui la verrouille définitivement sur la portion 26 de l'étui 5. Le chanfrein 52 est quant à lui destiné à permettre l'engagement de la tête 17 dans le logement défini par la paroi 46, jusqu'à encliquetage, en fin d'utilisation, du rebord 24 derrière des nervures 53 aménagées sur les zones de la bague 6 qui délimitent les bords proximaux des fenêtres 51. Cet encliquetage assure le verrouillage de la tige 4 dans la position montrée sur les figures 10 et 11. La portion 17a de la tête 17 assure le maintien des dents 33 dans une position radiale extérieure, qui permet d'assurer le verrouillage de la bague 6.

Les parties de maintien 47 sont diamétralement opposées et présentent chacune une forme de fourche, c'est-à-dire comprennent chacune une paire de bras 55 et un corps de liaison 56 reliant ces bras 55 à la bague 6.

Les bras 55 sont conformés, notamment arrondis dans l'exemple représenté, de manière à pouvoir entourer partiellement le corps de seringue 2. Ainsi que le montre plus particulièrement la figure 6, ils comprennent des chanfreins 57 aménagés sur leur côté proximal, par lequel un corps de seringue 2 est destiné à être introduit au travers de la bague 6.

Chaque bras 55 comprend également, au niveau de son extrémité libre, un bossage 58 faisant saillie radialement vers l'intérieur. Il peut comprendre plusieurs de tels bossages.

Les bras 55 peuvent être inclinés vers la bague 6 de manière à plaquer la collerette 12 contre les parties d'assemblage 48.

Le corps de liaison 56 de chaque partie de maintien 47 présente une forme coudée, c'est-à-dire comprend une partie de base, reliée au reste de la bague 6 et disposée sensiblement parallèlement à l'axe de cette bague 6, et une partie intermédiaire, reliée auxdits bras 55 et disposée sensiblement perpendiculairement à l'axe de la bague 6.

Les parties de base sont disposées sur le même diamètre que celui selon lequel font saillie les ailettes 45.

Ainsi que le montre la figure 5, les corps de liaison 56 sont conformés de telle sorte qu'après assemblage de la bague 6 sur l'étui 5, lesdites parties intermédiaires sont engagées à coulissement dans les saignées 32 et que lesdites parties de base font saillie par rapport à la paroi de l'étui 5, grâce aux méplats 26a de la portion 26.

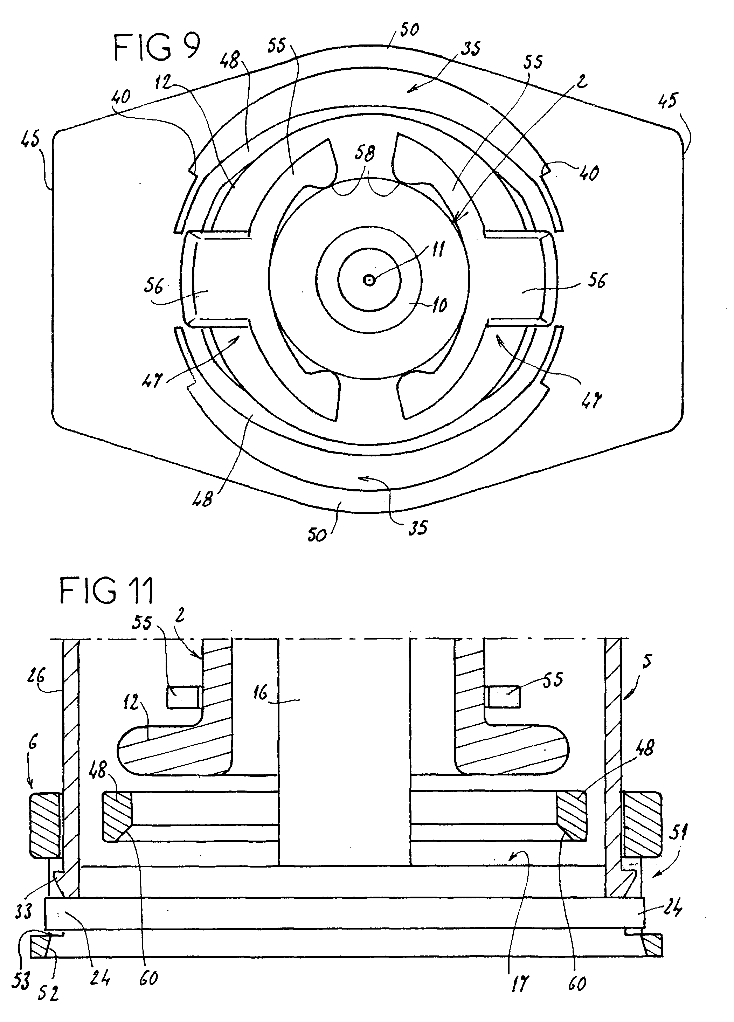

Comme cela se comprend en référence aux figures 8 et 9, les bras 55 sont destinés à recevoir entre eux et à enserrer le corps de seringue 2, et peuvent être déformés élastiquement de manière à pouvoir recevoir entre eux, et enserrer, des corps de seringue 2 de diamètres extérieurs différents, en particulier un corps 2 dit "0,5 ml" comme montré sur la figure 8 et un corps 2 dit "1 ml" comme montré sur la figure 9.

Les chanfreins 57 permettent de faciliter l'engagement d'un corps de seringue 2 entre ces bras, et les bossages 58 permettent d'assurer l'immobilisation et le centrage d'un corps de seringue 2 de diamètre réduit.

La relative souplesse des corps de liaison 56 dans le sens radial de la bague 6 permet de placer les bras 55 selon plusieurs positions radiales distinctes, et donc d'assurer un bon serrage d'un corps de seringue 2, sur une large gamme de diamètres de ceux-ci. Ces bras 55 permettent également, particulièrement lorsqu'ils sont inclinés vers la bague 6, de plaquer la collerette 12 contre les parties d'assemblage 48.

En outre, la présence desdites parties de base des corps de liaison 56 permet d'écarter les doigts de l'utilisateur par rapport à la paroi de l'étui 5, comme le montre la figure 5, éliminant ainsi tout risque de friction susceptible de freiner le mouvement d'activation du système de verrouillage, voire de compromettre ce verrouillage.

Les pattes 30 et les bossages 31 permettent quant à eux, ainsi que cela se déduit de la figure 4, de caler latéralement des corps de seringue 2 de diamètres extérieurs différents, les pattes 30 se déformant au besoin vers l'extérieur de manière à permettre l'effacement adéquat des bossages 31.

Les deux parties d'assemblage 48 sont délimitées par l'ouverture centrale de la bague 6 d'une part et par les lumières 35 d'autre part. Elles ont une épaisseur telle qu'elles sont déformables élastiquement dans le sens radial, cette déformabilité étant rendue importante grâce aux fentes 41.

Ces parties 48 constituent par exemple un anneau de forme ovale, relié, au niveau de leurs zones périphériques, les plus éloignées l'une de l'autre, à la bague 6, à proximité des parties de base des corps de liaison 56. Ces zones périphériques sont situées l'une par rapport à l'autre à une distance supérieure au diamètre externe de la collerette 12 du corps 2 tandis que les zones médianes de cet anneau, les plus proches l'une de l'autre, sont situées l'une par rapport à l'autre, à l'état non déformé de l'anneau, à une distance inférieure au diamètre extérieur de cette collerette 12.

La figure 11 montre que la partie proximale 26 de l'étui 5 présente un diamètre tel qu'il existe un espace entre la paroi de cette partie proximale 26 et lesdites zones médianes dudit anneau, cet espace permettant la déformation radiale de ces zones médianes.

En outre, des chanfreins 60 sont aménagés dans ces mêmes zones médianes.

Ainsi que cela se déduit des figures 6, 8, 9 et 11, la collerette 12 est destinée à être engagée au travers de l'anneau ovale précité jusqu'au-delà des parties 48. Les chanfreins 60 de ces parties 48 permettent l'effacement desdites zones médianes pour autoriser le passage de la collerette 12. Le rappel élastique de ces parties 48 dans leur forme d'origine permet le verrouillage de la collerette 12 au-delà de ces parties 48.

Une autre forme de réalisation peut consister en ce que les parties d'assemblage 48 constituent un anneau sensiblement circulaire avec un ou plusieurs bossages dans les zones médianes, les plus déformables.

Le corps 2 est alors parfaitement assemblé à la bague 6, sans aucun jeu radial et avec un jeu axial très limité.

En pratique, comme cela se comprend par comparaison des figures 2 et 10, l'utilisateur réalise l'injection en exerçant des pressions antagonistes sur les ailettes 45 et sur la tête 17, au moyen respectivement de deux doigts, en particulier le majeur et l'index, d'une part, et d'un troisième doigt, en particulier le pouce, d'autre part.

A la fin de l'injection, le piston 3 vient en butée contre le fond du corps de seringue 2 ou la tête 17 vient en butée contre l'étui 5 ; lesdites forces antagonistes peuvent alors être exercées de manière plus intense, jusqu'à rupture des ponts 20. Il en résulte un déplacement relatif brusque de la bague 6, et donc du corps de seringue 2, par rapport à la tête 17, susceptible d'amener la venue de la bague 6 et de la tête 17 dans une position de sécurité, montrée sur la figure 10, dans laquelle l'aiguille 11 est complètement rétractée dans l'étui 5.

Ce mouvement brusque est réalisé dans le prolongement du geste d'utilisation de la seringue 1 pour l'injection. Le caractère brusque de ce mouvement, associé à l'écartement des doigts de l'utilisateur par rapport à l'étui 5 résultant de la présence desdites parties de base des corps de liaison 56, assure que la position de sécurité est effectivement atteinte dans tous les cas.

Le verrouillage de la tête 17 est réalisé automatiquement en fin de ce mouvement brusque, par engagement du rebord 24 derrière les nervures 53. L'engagement de la portion 17a de la tête 17 dans l'extrémité proximale de l'étui 5, comme le montre la figure 11, élimine, ou tout au moins rend très difficile, toute tentative de déverrouillage de la tige 4.

Ainsi qu'il apparaît de ce qui précède, l'invention fournit un dispositif ayant pour avantage essentiel de procurer à l'utilisateur une sécurité parfaite, ou tout au moins renforcée, contre le risque de piqûre ou de coupure accidentelle après utilisation, en éliminant toute nécessité d'action distincte et séparée pour le déplacement de l'étui de protection en position de sécurité, et en éliminant toute possibilité de contrôle de ce déplacement.

Il va de soi que l'invention n'est pas limitée à la forme de réalisation décrite ci-dessus à titre d'exemple mais qu'elle en embrasse au contraire toutes les variantes de réalisation couvertes par les revendications ci-annexées.

## Revendications

1. Dispositif à usage médical, notamment seringue, à sécurité renforcée, comprenant :
(i) un organe acéré ou tranchant (11), notamment une aiguille, destiné à être introduit dans un tissu du patient ou à être mis en contact avec un fluide corporel du patient, et donc à être éventuellement contaminé par ce tissu ou ce fluide corporel ;
(ii) un étui de protection (5), engagé autour d'une pièce (2) du dispositif, notamment un corps de seringue, comprenant ledit organe acéré ou tranchant (11) ; cet étui (5) et cette pièce (2) sont mobiles l'un par rapport à l'autre entre une position d'utilisation du dispositif (1), dans laquelle ledit organe acéré ou tranchant (11) est exposé, et une position de sécurité, dans laquelle cet organe est entouré par l'étui (5), de manière à prévenir tout risque de piqûre ou de coupure après utilisation, et donc de contamination éventuelle, de l'utilisateur par cet organe (11 ) ; et
(iii) des moyens de déplacement (6) permettant, notamment par action manuelle, de déplacer ladite pièce (2) et ledit étui (5) l'un par rapport à l'autre entre lesdites positions d'utilisation et de sécurité ;
- l'étui de protection (5) comprenant au moins une lumière ou saignée longitudinale (32) ;
- la pièce (2) comportant l'organe acéré ou tranchant (11) étant reliée à une première pièce (6) de prise d'appui pouvant coulisser par rapport à l'étui de protection (5), cette première pièce de prise d'appui (6) présentant au moins une partie (45) de prise d'appui, engagée au travers de ladite lumière ou saignée (32) et faisant saillie au-delà de l'étui de protection (5), qui sert à la prise d'appui de deux doigts, notamment l'index et le majeur, de la main de l'utilisateur ;
- le dispositif (1) comprenant une deuxième pièce de prise d'appui (4), notamment une tige de piston, présentant une partie de prise d'appui (17) pour un troisième doigt, notamment le pouce, de l'utilisateur, et
- le dispositif (1) comprenant des moyens ruptibles (20), propres à se rompre au-delà d'un seuil de forces antagonistes exercées sur lesdites parties de prise d'appui (45, 17) respectives desdites première et deuxième pièces de prise d'appui (6, 4), la rupture de ces moyens (20) générant un mouvement relatif brusque de ladite pièce (2) comportant l'organe acéré ou tranchant (11) et dudit étui de protection (5), tel que cette pièce (2) et cet étui (5) sont amenés en position de sécurité à l'issue de ce mouvement brusque ;
dispositif (1) **caractérisé en ce qu'**il comprend des moyens de verrouillage (53, 24) de la pièce (2) comportant l'organe acéré ou tranchant (11 ) et de l'étui de protection (5) en position de sécurité.

2. Dispositif selon la revendication 1, **caractérisé en ce qu'**il forme une seringue (1 ) et **en ce que** :
- ladite pièce comportant l'organe acéré ou tranchant (11) est formée par un corps de seringue (2) présentant une aiguille sur son extrémité distale et un moyen d'assemblage (12), tel qu'une collerette, sur son extrémité proximale ;
- ladite première pièce de prise d'appui est formée par une bague (6) comprenant une partie logée à l'intérieur de l'étui de protection (5), munie de moyens d'assemblage (48, 55) complémentaires de celui du corps de seringue (2) pour assurer une liaison axiale entre cette bague (6) et ce corps de seringue (2), et une partie dépassant de l'étui, comprenant deux saillies (45) en forme d'ailettes, par exemple diamétralement opposées, formant lesdites parties de prise d'appui, destinées à recevoir deux doigts, notamment l'index et le majeur, de la main de l'utilisateur ;
- ladite deuxième pièce de prise d'appui est formée par la tige de piston (4) de la seringue (1) ; cette tige de piston (4) comprend deux parties télescopiques (15, 16), dont une, distale, est reliée au piston (3) de la seringue, et dont l'autre, proximale, comporte une tête (17) formant ladite partie de prise d'appui pour un troisième doigt, notamment le pouce, de l'utilisateur, et
- lesdits moyens ruptibles sont constitués par au moins un pont de matière (20) reliant les deux parties télescopiques (15, 16) de la tige de piston (4), ledit seuil de force de rupture de ce ou ces ponts (20) étant supérieur à la force nécessaire au déplacement du piston (3) mais inférieur aux forces antagonistes qu'un utilisateur est susceptible d'exercer manuellement sur lesdites saillies (45) et ladite tête (17).

3. Dispositif selon la revendication 2, **caractérisé en ce que** la tige (4) du piston (3) présente une longueur telle que la tête de piston (17) n'est pas en butée contre le bord proximal de l'étui (5) lorsque le piston (3) est en butée contre le fond du corps de seringue (2).

4. Dispositif selon l'une des revendications 1 à 3, **caractérisé en ce que** les moyens de verrouillage (53, 24) sont du type à enclenchement, notamment à encliquetage, et sont positionnés de sorte que le verrouillage se produit automatiquement au moment où la pièce (2) comportant l'organe acéré ou tranchant (11) et l'étui de protection (5) atteignent ladite position de sécurité.

5. Dispositif selon la revendication 4, **caractérisé en ce que**:
- la bague (6) présente une paroi périphérique (46) définissant un logement propre à recevoir étroitement, en fin d'injection, la tête (17) que comprend la tige (4) d'actionnement du piston (3) de la seringue (1 ) ;
- des moyens de verrouillage (53) sont aménagés dans ledit logement ; et
- la tête (17) de la tige de piston (4) comprend des moyens de verrouillage (24) propres à venir en prise avec les moyens de verrouillage (53) de la bague (6) lorsque cette tête (17) est reçue dans ledit logement.

6. Dispositif selon la revendication 5, **caractérisé en ce que** le bord proximal de ladite paroi périphérique (46) présente un chanfrein (52) pour faciliter l'engagement de ladite tête (17) dans ledit logement.

7. Dispositif selon la revendication 5 ou la revendication 6, **caractérisé en ce que** les moyens de verrouillage que comprend la paroi périphérique (46) sont constitués par au moins une nervure (53) aménagée dans cette paroi (46), et **en ce que** les moyens de verrouillage que comprend ladite tête (17) sont constitués par un rebord (24) correspondant faisant saillie radialement de cette tête (17) vers l'extérieur, propre à être engagé et verrouillé par encliquetage derrière cette ou ces nervures (53).

8. Dispositif selon l'une des revendications 5 à 7, **caractérisé en ce que** :
- l'étui (5) comprend une portion proximale (26) qui présente, au niveau de son bord proximal, deux dents d'encliquetage (33) à même d'assurer la rétention de la bague 6 sur cet étui (5) ; et
- la tête (17) de la tige de piston (4) comprend un redan périphérique (23), qui délimite une portion cylindrique (17a) de la tête (17) de la tige de piston (4) et un rebord (24) faisant saillie radialement vers l'extérieur, le diamètre de cette portion cylindrique (17a) étant tel que cette portion cylindrique (17a) peut être engagée avec ajustement dans la portion proximale (26) de l'étui (5) en fin d'injection, de manière à assurer le maintien des dents (33) dans une position radiale extérieure, de verrouillage de la bague (6).

9. Dispositif selon l'une des revendications 2 à 8, **caractérisé en ce que**
- la partie de la bague (6) située à l'intérieur de l'étui (5) comprend deux parties de maintien (47) en forme de fourche, c'est-à-dire comprenant chacune une paire de bras (55) et un corps de liaison (56) reliant ces bras (55) à la bague (6), les bras (55) de chaque fourche étant conformés de manière à pouvoir entourer partiellement le corps de seringue (2) ; et
- lesdits bras (55) sont réalisés en un matériau présentant un degré de souplesse élastique tel qu'ils peuvent être déformés élastiquement de manière à pouvoir recevoir entre eux, et enserrer, des corps de seringue (2) de diamètres extérieurs différents.

10. Dispositif selon la revendication 9, **caractérisé en ce que** chacun des bras (55) d'une partie de maintien (47) comprend au moins un bossage (58) faisant saillie radialement vers l'intérieur.

11. Dispositif selon la revendication 9 ou la revendication 10, **caractérisé en ce que** le corps de liaison (56) de chaque partie de maintien (47) présente une forme coudée, c'est-à-dire comprend une partie de base, reliée au reste de la bague (6) et disposée sensiblement parallèlement à l'axe de cette bague (6), et une partie intermédiaire, reliée auxdits bras (55) et disposée sensiblement perpendiculairement à l'axe de la bague (6), lesdites parties de base étant disposées sur le même diamètre que celui selon lequel font saillie les parties (45) en forme d'ailettes.

12. Dispositif selon l'une des revendications 9 à 11, **caractérisé en ce qu'**au moins une desdites parties de maintien (47) présente une structure flexible, notamment au niveau de son corps de liaison (56), de manière à faciliter l'assemblage du corps de seringue à la bague (6).

13. Dispositif selon l'une des revendications 9 à 12, **caractérisé en ce que** lesdites parties de maintien (47) sont conformées de manière à plaquer les moyens d'assemblage (12) du corps (2) de seringue contre la bague (6) ou une portion déterminée (48) de celle-ci.

14. Dispositif selon l'une des revendications 2 à 13, **caractérisé en ce que** l'étui de protection (5) présente des moyens de calage (30, 31) permettant de caler latéralement un corps de seringue (2) mis en place dans cet étui (5), ces moyens de calage (30, 31) étant mobiles radialement de manière à pouvoir caler des corps de seringue (2) de diamètres extérieurs différents.

15. Dispositif selon l'une des revendications 9 à 14, **caractérisé en ce que** :
- le corps de seringue (2) présente une collerette proximale (12) pour son assemblage à la bague (6) ;
- la partie de la bague (6) située à l'intérieur de l'étui (5) comprend les parties de maintien (47) précitées, et
- la bague (6) comprend deux parties d'assemblage (48) de forme courbe, disposées de manière à former substantiellement un anneau ; ces parties d'assemblage (48) sont reliées à la bague (6) au niveau de zones périphériques et sont réalisées en un matériau présentant un degré de souplesse élastique ; les zones périphériques de ces parties d'assemblage (48) sont situées l'une par rapport à l'autre à une distance supérieure au diamètre externe de ladite collerette (12) tandis que les zones médianes de ces parties d'assemblage (48) sont situées l'une par rapport à l'autre, à l'état non déformé de ces parties d'assemblage (48), à une distance inférieure au diamètre extérieur de la collerette (12) ; ces zones médianes présentent, du côté de la bague (6) par lequel le corps de seringue (2) est engagé au travers des parties d'assemblage (48), des chanfreins (60) conformés pour permettre l'effacement radial de ces zones médianes au moment de cet engagement.

16. Dispositif selon la revendication 15, **caractérisé en ce que** la bague (6) comprend deux lumières (35) de forme arquée qui délimitent lesdites parties d'assemblage (48) et reçoivent l'étui (5) en elles, ces lumières (35) présentant des épaulements de calage (40) au niveau de leurs extrémités longitudinales, qui permettent de caler en rotation la bague (6) par rapport à l'étui (5).

17. Dispositif selon l'une des revendications 2 à 16, **caractérisé en ce que** la partie distale (15) de la tige de piston (4) se prolonge, du côté proximal, au-delà du ou desdits ponts (20), et **en ce que** cette prolongation (21 ) présente un diamètre extérieur légèrement inférieur au diamètre intérieur de la partie proximale (16) de cette tige de piston (4).

18. Dispositif à usage médical, avec sécurité, comprenant
(a) un sous-ensemble du type seringue selon l'une des revendications 1 à 17, comprenant le corps (2) allongé selon un axe de référence ; l'aiguille (11) disposée axialement, communiquant avec l'intérieur dudit corps, à l'extrémité distale de ce dernier ; le piston (3) déplaçable à l'intérieur dudit corps jusqu'à une position distale en butée ; et la tige (4) de piston émergeant à l'extrémité proximale dudit corps (2), avec la partie proximale de prise d'appui (17) ;
(b) un sous-ensemble de sécurité, comprenant l'étui de protection (5) monté autour du corps (2), déplaçable axialement par rapport à ce dernier entre deux positions, à savoir une position d'exposition dans laquelle l'aiguille (11) est exposée et une position de sécurité dans laquelle l'aiguille (11) est entièrement contenue à l'intérieur dudit étui de protection (5);
dispositif **caractérisé en ce que**, en combinaison :
(i) l'étui de protection (5) est prolongé par une portion proximale (26) au-delà de l'extrémité proximale du corps (2) dans la position d'exposition dudit étui de protection, la tige (4) de piston émergeant de l'extrémité proximale de ladite portion proximale (26) ;
(ii) la bague d'actionnement (6) s'étend radialement à partir de l'extrémité proximale du corps (2), en étant solidaire de cette dernière, et comporte une partie externe (45) de prise d'appui, de part et d'autre du corps (2) ;
(iii) des moyens (41) de coulissement axial entre la bague d'actionnement (6) et la portion proximale (26) de l'étui de protection (5), ladite portion proximale étant montée librement en translation au travers de la bague (6) ;
(iv) des moyens (20) de rétraction axiale de la tige (4) de piston sur elle-même, par poussée axiale sur la partie proximale de prise d'appui (17) lorsque le piston (3) est dans sa position distale en butée, en sorte que ladite partie proximale de prise d'appui (17) vient en butée contre l'extrémité proximale de l'étui de protection (5), puis déplace ce dernier vers sa position de sécurité, toujours par poussée.

## Patentansprüche

1. Vorrichtung zur medizinischen Verwendung, insbesondere als Spritze, mit verbesserter Sicherheit, die folgendes aufweist:
(i) ein scharfes oder schneidendes Organ (11), insbesondere eine Nadel, zum Einführen in ein Gewebe des Patienten oder zum Inkonkaktbringen mit einer Körperflüssigkeit des Patienten, das daher gegebenenfalls durch dieses Gewebe bzw. diese Körperflüssigkeit kontaminiert wird;
(ii) eine Schutzhülse (5), die um einen Teil (2) der Vorrichtung, insbesondere einen Spritzenkörper, der das scharfe oder schneidende Organ (11) aufweist, angeschlossen ist; wobei die Hülse (5) und dieses Teil (2) relativ zueinander beweglich sind zwischen einer Verwendungsposition der Vorrichtung (1), in welcher das scharfe oder schneidende Organ (11) freiliegt, und einer Sicherheitsposition, in welcher das Organ von der Hülse (5) umgeben ist, um jegliches Risiko einer Stich- oder Schnittverletzung und somit einer eventuellen Kontaminierung des Anwenders durch das Organ (11) nach der Verwendung zu vermeiden; und
(iii) eine Verschiebungseinrichtung (6), die es ermöglicht, insbesondere durch manuelle Betätigung das Teil (2) und die Hülse (5) relativ zueinander zwischen der Verwendungs- und der Sicherheitsposition zu verschieben;
- wobei die Schutzhülse (5) mindestens eine Aussparung oder einen Schlitz (32) aufweist, die bzw. der sich in Längsrichtung erstreckt;
- wobei das Teil (2), welches das scharfe oder schneidende Organ (11) aufweist, mit einem ersten Abstützelement (6) verbunden ist, das bezüglich der Schutzhülse (5) gleitverschieblich ist, wobei dieses erste Abstützelement (6) mindestens ein Abstützteil (45) aufweist, das über die Aussparung bzw. den Schlitz (32) hinweg in Eingriff steht und über die Schutzhülse vorsteht (5) und das zum Abstützen von zwei Fingern, insbesondere des Zeigefingers und des Mittelfingers, der Hand des Anwenders dient;
- wobei die Vorrichtung (1) ein zweites Abstützelement (4), insbesondere eine Kolbenstange, mit einem Abstützteil (17) für einen dritten Finger, insbesondere den Daumen, des Anwenders, aufweist, und
- wobei die Vorrichtung (1) Sollbrucheinrichtungen (20) aufweist, die dazu geeignet sind, oberhalb eines Grenzwertes von einander entgegenwirkenden Kräften, die auf die jeweiligen Abstützteile (45, 17) des ersten und des zweiten Abstützelementes (6, 4) ausgeübt werden, abzubrechen, wobei das Abbrechen dieser Einrichtungen (20) eine abrupte Relativbewegung des Teils (2), welches das scharfe oder schneidende Organ (11) aufweist, und der Schutzhülse (5) erzeugt, so daß das Teil (2) und die Hülse (5) bei Beendigung der abrupten Bewegung in die Sicherheitsposition gebracht werden;
wobei die Vorrichtung (1) **dadurch gekennzeichnet ist,**
**daß** sie Einrichtungen (53, 24) zum Verriegeln des Teils (2), welches das scharfe oder schneidende Organ (11) aufweist, und der Schutzhülse (5) in der Sicherheitsposition aufweist.

2. Vorrichtung nach Anspruch 1,
**dadurch gekennzeichnet,**
**daß** sie eine Spritze (1) bildet, sowie dadurch, daß:
- das Teil, welches das scharfe oder schneidende Organ (11) aufweist, von einem Spritzenkörper (2) gebildet ist, der an seinem distalen Ende eine Nadel und an seinem proximalen Ende eine Montageeinrichtung (12), wie etwa einen Kragen aufweist;
- das erste Abstützelement von einem Ring (6) gebildet ist, der ein Teil aufweist, das im Inneren der Schutzhülse (5) aufgenommen ist, der mit Montageeinrichtungen (48, 55) versehen ist, die zu demjenigen des Spritzenkörpers (2) komplementär sind, zum Herstellen einer axialen Verbindung zwischen dem Ring (6) und dem Spritzenkörper (2), und der ein Teil aufweist, das über die Hülse vorsteht, und der zwei flügelförmige Vorsprünge (45) aufweist, die einander beispielsweise diametral gegenüberliegen und die Abstützteile bilden, die dazu vorgesehen sind, zwei Finger, insbesondere den Zeigefinger und den Mittelfinger, der Hand des Anwenders aufzunehmen;
- das zweite Abstützelement von der Kolbenstange (4) der Spritze (1) gebildet ist; wobei die Kolbenstange (4) zwei teleskopierbare Teile (15, 16) aufweist, von denen das eine, distale, mit dem Kolben (3) der Spritze verbunden ist, und das andere, proximale, einen Kopf (17) aufweist, der das Abstützelement für einen dritten Finger, insbesondere den Daumen, des Anwenders bildet, und
- die Sollbrucheinrichtungen aus mindestens einer Materialbrücke (20) bestehen, welche die beiden teleskopierbaren Teile (15, 16) der Kolbenstange (4) verbindet, wobei der Grenzwert der Kraft zum Brechen dieser Brücke oder Brücken (20) größer als die zum Verschieben des Kolbens (3) nötige Kraft ist, aber kleiner ist als die einander entgegenwirkenden Kräfte, die ein Anwender manuell auf die Vorsprünge (45) und den Kopf (17) ausüben kann.

3. Vorrichtung nach Anspruch 2,
**dadurch gekennzeichnet,**
**daß** die Stange (4) des Kolbens (3) eine solche Länge aufweist, daß der Kolbenkopf (17) nicht am proximalen Rand der Hülse (5) anliegt, wenn der Kolben (3) am Boden des Spritzenkörpers (2) anliegt.

4. Vorrichtung nach einem der Ansprüche 1 bis 3,
**dadurch gekennzeichnet,**
**daß** die Verriegelungseinrichtungen (53, 24) vom Einschnapp-Typ, insbesondere solche durch Einrasten sind und derart positioniert sind, daß das Verriegeln selbsttätig in dem Moment stattfindet, in dem das Teil (2), welches das scharfe oder schneidende Organ (11) aufweist, und die Schutzhülse (5) die Sicherheitsposition erreichen.

5. Vorrichtung nach Anspruch 4,
**dadurch gekennzeichnet,**
- **daß** der Ring (6) eine Umfangswand (46) aufweist, welche eine Aufnahme bildet, die in der Lage ist, bei Beendigung einer Injektion den Kopf (17), welchen die Stange (4) zum Betätigen des Kolbens (3) der Spritze (1) aufweist, eng anliegend aufzunehmen;
- **daß** Verriegelungseinrichtungen (53) in der Aufnahme vorgesehen sind; und
- **daß** der Kopf (17) der Kolbenstange (4) Verriegelungseinrichtungen (24) aufweist, die in der Lage sind, in Eingriff mit den Verriegelungseinrichtungen (53) des Rings (6) zu treten, wenn der Kopf (17) in der Aufnahme aufgenommen ist.

6. Vorrichtung nach Anspruch 5,
**dadurch gekennzeichnet,**
**daß** der proximale Rand der Umfangswand (46) eine Schrägung (52) aufweist, um den Eingriff des Kopfes (17) in der Aufnahme zu erleichtern.

7. Vorrichtung nach Anspruch 5 oder Anspruch 6,
**dadurch gekennzeichnet,**
**daß** die Verriegelungseinrichtungen, welche die Umfangswand (46) aufweist, aus mindestens einer Rippe (53) bestehen, die an dieser Wand (46) vorgesehen ist, sowie dadurch, daß die Verriegelungseinrichtungen, die der Kopf (17) aufweist, aus einem entsprechenden Umfangsrand (24) bestehen, der radial von diesem Kopf (17) nach außen vorsteht, so daß er durch Einrasten hinter dieser Rippe bzw. diesen Rippen (53) in Eingriff gebracht und verriegelt werden kann.

8. Vorrichtung nach einem der Ansprüche 5 bis 7,
**dadurch gekennzeichnet,**
- **daß** die Hülse (5) einen proximalen Bereich (26) aufweist, der auf Höhe seiner proximalen Kante zwei Rastzähne (33) aufweist, die in der Lage sind, das Festhalten des Rings (6) an dieser Hülse (5) zu bewirken; und
- **daß** der Kopf (17) der Kolbenstange (4) einen umfangsmäßigen Absatz (23), der einen zylindrischen Bereich (17a) des Kopfes (17) der Kolbenstange (4) begrenzt, und einen radial nach außen vorstehenden Umfangsrand (24) aufweist, wobei der Durchmesser dieses zylindrischen Bereichs (17a) derart ausgebildet ist, daß der zylindrische Bereich (17a) bei Beendigung einer Injektion mit Passung im proximalen Teil (26) der Hülse (5) in Eingriff gebracht werden kann, so daß bewirkt wird, daß die Vorsprünge (33) in einer äußeren radialen Position zur Verriegelung des Rings (6) gehalten werden.

9. Vorrichtung nach einem der Ansprüche 2 bis 8,
**dadurch gekennzeichnet,**
- **daß** der im Inneren der Hülse (5) befindliche Teil des Rings (6) zwei Halteteile (47) aufweist, die eine Gabelform besitzen, d.h. die jeweils ein Paar von Armen (55) und einen Verbindungskörper (56) aufweisen, welcher diese Arme (55) mit dem Ring (6) verbindet, wobei die Arme (55) einer jeden Gabel derart formmäßig angepaßt sind, daß sie den Spritzenkörper (2) teilweise umgeben können; und
- **daß** die Arme (55) aus einem Material hergestellt sind, das einen solchen Grad von elastischer Nachgiebigkeit besitzt, daß sie elastisch verformt werden können, so daß sie zwischeneinander Spritzenkörper (2) mit unterschiedlichen Außendurchmessern aufnehmen und einspannen können.

10. Vorrichtung nach Anspruch 9,
**dadurch gekennzeichnet,**
**daß** jeder der Arme (55) eines Halteteils (47) mindestens eine Verdickung (58) aufweist, die radial nach innen vorsteht.

11. Vorrichtung nach Anspruch 9 oder Anspruch 10,
**dadurch gekennzeichnet,**
**daß** der Verbindungskörper (56) eines jeden Halteteils (47) eine gekrümmte Form besitzt, d. h. ein Basisteil, das mit dem Rest des Rings (6) verbunden ist und im wesentlichen parallel zur Achse des Rings (6) ist, und ein Zwischenteil aufweist, das mit den Armen (55) verbunden ist und im wesentlichen senkrecht zur Achse des Rings (6) steht, wobei die Basisteile auf dem gleichen Durchmesser wie demjenigen angeordnet sind, entlang dem die flügelförmigen Teile (45) vorstehen.

12. Vorrichtung nach einem der Ansprüche 9 bis 11,
**dadurch gekennzeichnet,**
**daß** mindestens eines der Halteteile (47), insbesondere auf der Höhe seines Verbindungskörpers (56), eine flexible Struktur aufweist, so daß es die Anbringung des Spritzenkörpers am Ring (6) erleichtert.

13. Vorrichtung nach einem der Ansprüche 9 bis 12,
**dadurch gekennzeichnet,**
**daß** die Halteteile (47) derart formmäßig angepaßt sind, daß sie die Montageeinrichtungen (12) des Körpers (2) der Spritze gegen den Ring (6) oder einen bestimmten Bereich (48) davon andrücken.

14. Vorrichtung nach einem der Ansprüche 2 bis 13,
**dadurch gekennzeichnet,**
**daß** die Schutzhülse (5) Blockiereinrichtungen (30, 31) aufweist, welche es ermöglichen, einen in dieser Hülse (5) angeordneten Spritzenkörper (2) seitlich zu blockieren, wobei die Blockiereinrichtungen (30, 31) radial beweglich sind, so daß sie in der Lage sind, Spritzenkörper (2) mit unterschiedlichen Außendurchmessern zu blockieren.

15. Vorrichtung nach einem der Ansprüche 9 bis 14,
**dadurch gekennzeichnet, daß**:
- der Spritzenkörper (2) einen proximalen Kragen (12) für seine Anbringung am Ring (6) aufweist;
- der im Inneren der Hülse (5) befindliche Teil des Rings (6) die genannten Halteteile (47) aufweist, und
- der Ring (6) zwei Montageteile (48) mit einer gekrümmten Form aufweist, die so angeordnet sind, daß sie im wesentlichen einen Ring bilden; wobei diese Montageteile (48) auf der Höhe von Umfangszonen mit dem Ring (6) verbunden und aus einem Material hergestellt sind, das einen Grad an elastischer Nachgiebigkeit besitzt; wobei die Umfangszonen dieser Montageteile (48) sich relativ zueinander in einem Abstand befinden, der größer als der Außendurchmesser des Kragens (12) ist, während sich die Mittelbereiche dieser Montageteile (48) im nicht verformten Zustand dieser Montageteile (48) relativ zueinander in einem Abstand befinden, der kleiner als der Außendurchmesser des Kragens (12) ist; wobei diese Mittelbereiche auf der Seite des Rings (6), mit dem der Spritzenkörper (2) über die Montageteile (48) hinweg in Eingriff gebracht wird, Abschrägungen (60) aufweisen, die derart formmäßig angepaßt sind, daß sie das radiale Überwinden dieser Mittelbereiche im Augenblick dieses Eingriffs ermöglichen.

16. Vorrichtung nach Anspruch 15,
**dadurch gekennzeichnet,**
**daß** der Ring (6) zwei bogenförmige Aussparungen (35) aufweist, welche die Montageteile (48) begrenzen und die Hülse (5) in sich aufnehmen, wobei diese Aussparungen (35) auf der Höhe ihrer Enden in Längsrichtung Blockierschultern (40) aufweisen, die es ermöglichen, den Ring (6) relativ zur Hülse (5) rotationsmäßig zu blockieren.

17. Vorrichtung nach einem der Ansprüche 2 bis 16,
**dadurch gekennzeichnet,**
**daß** das distale Teil (15) der Kolbenstange (4) auf der proximalen Seite über die Brücke oder die Brücken (20) hinaus verlängert ist, sowie dadurch, daß diese Verlängerung (21) einen Außendurchmesser aufweist, der geringfügig kleiner als der Innendurchmesser des proximalen Teils (16) der Kolbenstange (4) ist.

18. Vorrichtung zur medizinischen Verwendung mit Sicherheit,
die folgendes aufweist:
(a) eine Untereinheit vom Spritzen-Typ nach einem der Ansprüche 1 bis 17, welche folgendes aufweist:
den Körper (2) mit einer langgestreckten Form gemäß einer Bezugsachse; die axial angeordnete Nadel (11), die mit dem Inneren des Körpers an dessen distalem Ende in Verbindung steht; den Kolben (3), der im Inneren des Körpers bis zu einer distalen Anschlagposition verschiebbar ist; und die Stange (4) des Kolbens, die am proximalen Ende des Körpers (2) austritt, mit dem proximalen Abstützteil (17);
(b) eine Sicherheits-Untereinheit, welche die um den Körper (2) herum montierte Schutzhülse (5) aufweist und relativ zu diesem Körper zwischen zwei Positionen axial verschiebbar ist, nämlich einer Freigabeposition, in welcher die Nadel (11) freiliegt, und einer Sicherheitsposition, in welcher die Nadel (11) vollständig im Inneren der Schutzhülse (5) enthalten ist;
wobei die Vorrichtung **dadurch gekennzeichnet ist,**
**daß** in Kombination:
(i) die Schutzhülse (5) in der Freigabeposition der Schutzhülse durch ein proximales Teil (26) über das proximale Ende des Körpers (2) hinaus verlängert ist, wobei die Stange (4) des Kolbens aus dem proximalen Ende des proximalen Teils (26) austritt;
(ii) der Betätigungsring (6) sich in radialer Richtung vom proximalen Ende des Körpers (2) erstreckt, dabei einstückig mit diesem ausgebildet ist und beidseitig vom Körper (2) ein äußeres Abstützteil (45) aufweist;
(iii) Einrichtungen (41) zum gleitenden axialen Verschieben zwischen dem Betätigungsring (6) und dem proximalen Teil (26) der Schutzhülse (5), wobei das proximale Teil frei für eine Translationsbewegung über den Ring (6) verschiebbar montiert ist;
(iv) Einrichtungen (20) zum axialen Zurückziehen der Stange (4) des Kolbens auf sich selbst durch axialen Druck auf das proximale Abstützteil (17), wenn sich der Kolben (3) in seiner distalen Anschlagposition befindet, so daß das proximale Abstützteil (17) gegen das proximale Ende der Schutzhülse (5) anliegt und letztere dann durch fortgesetzten Druck in ihre Sicherheitsposition hin verschiebt.

## Claims

1. A device for medical use, in particular a syringe, with improved safety, comprising:
(i) a sharp or sharp-edged member (11), in particular a needle, intended to be introduced into the patient's tissue or to be brought into contact with a body fluid of the patient, and thus to be contaminated possibly by this tissue or by this body fluid;
(ii) a protective casing (5) engaged around a piece (2) of the device, in particular a syringe body, comprising said sharp or sharp-edged member (11); this casing (5) and this piece (2) can move relative to one another between a use position of the device (1), in which said sharp or sharp-edged member (11) is exposed, and a safety position, in which this member is surrounded by the casing (5), in such a way as to prevent any risk of stick injuries or cuts after use, and thus of possible contamination of the user by this member (11); and
(iii) displacement means (6) which make it possible, in particular by manual activation, to displace said piece (2) and said casing (5) relative to one another between said use position and safety position;
- the protective casing (5) comprising at least one longitudinal slot or cutting (32);
- the piece (2) having the sharp or sharp-edged member (11) being connected to a first support piece (6) which can slide relative to the protective casing (5), this first support piece (6) having at least one support part (45), engaged through said slot or cutting (32) and protruding beyond the protective casing (5), and which serves to support two fingers, in particular the index finger and the middle finger, of the user's hand;
- the device (1) comprising a second support piece (4), in particular a plunger rod, having a support part (17) for a third finger, in particular the thumb of the user, and
- the device (1) comprising breakable means (20) which are able to break above a threshold of antagonistic forces exerted on said respective support parts (45, 17) of said first and second support pieces (6, 4), the breaking of these means (20) generating a sudden relative movement of said piece (2), with the sharp or sharp-edged member (11), and of said protective casing (5), such that this piece (2) and this casing (5) are brought into the safety position at the end of this sudden movement;
which device (1) comprises locking means (53, 24) with which the piece (2), having the sharp or sharp-edged member (11), and the protective casing (5) are locked in the safety position.

2. The device as claimed in claim 1, wherein it forms a syringe (1) and:
- said piece having the sharp or sharp-edged member (11) is formed by a syringe body (2) having a needle on its distal end and a means of connection (12), such as a flange, on its proximal end;
- said first support piece is formed by a ring (6) comprising a part lodged inside the protective casing (5), equipped with means of connection (48, 55) complementing those of the syringe body (2) in order to ensure an axial connection between this ring (6) and this syringe body (2), and a part protruding from the casing, comprising two wing-shaped projections (45), for example diametrically opposed, forming said support parts and intended to receive two fingers, in particular the index finger and the middle finger, of the user's hand;
- said second support piece is formed by the plunger rod (4) of the syringe (1); this plunger rod (4) comprises two telescopic parts (15, 16), one of which, the distal part, is connected to the plunger (3) of the syringe, and the other of which, the proximal part, has a head (17) forming said support part for a third finger, in particular the thumb, of the user; and
- said breakable means consist of at least one bridge of material (20) connecting the two telescopic parts (15, 16) of the plunger rod (4), said threshold force for breaking this bridge or these bridges (20) being greater than the force needed to displace the plunger (3) but lower than the antagonistic forces which a user is likely to exert manually on said projections (45) and said head (17).

3. The device as claimed in claim 2, wherein the rod (4) of the plunger (3) has a length which is such that the plunger head (17) is not in abutment against the proximal edge of the casing (5) when the plunger (3) is in abutment against the bottom of the syringe body (2).

4. The device as claimed in one of claims 1 through 3, wherein the locking means (53, 24) are of the catch type, in particular with snap-fitting, and are positioned in such a way that the locking takes place automatically at the moment when the piece (2), with the sharp or sharp-edged member (11), and the protective casing (5) reach said safety position.

5. The device as claimed in claim 4, wherein
- the ring (6) has a peripheral wall (46) defining a seat which, at the end of injection, can tightly receive the head (17) of the actuation rod (4) of the plunger (3) of the syringe (1);
- locking means (53) are arranged in said seat; and
- the head (17) of the plunger rod (4) comprises locking means (24) which can engage with the locking means (53) of the ring (6) when this head (17) is received in said seat.

6. The device as claimed in claim 5, wherein the proximal edge of said peripheral wall (46) has a bevel (52) to make engagement of said head (17) in said seat easier.

7. The device as claimed in claim 5 or 6, wherein the locking means which the peripheral wall (46) comprises are made up of at least one rib (53) formed in this wall (46), and wherein the locking means which said head (17) comprises are made up of a corresponding shoulder (24) which protrudes radially outward from this head (17) and which is able to be engaged and locked by snapping behind this rib or ribs (53).

8. The device as claimed in one of claims 5 through 7, wherein:
- the casing (5) comprises a proximal portion (26) which has, at its proximal edge, two snap-fit teeth (33) which are able to retain the ring (6) on this casing (5); and
- the head (17) of the plunger rod (4) comprises a peripheral step (23) which delimits a cylindrical portion (17a) of the head (17) of the plunger rod (4), and a shoulder (24) protruding radially outward, the diameter of this cylindrical portion (17a) being such that this cylindrical portion (17a) can be engaged with precise fit in the proximal portion (26) of the casing (5) at the end of injection, in such a way as to hold the teeth (33) in an outer radial position of locking of the ring (6).

9. The device as claimed in one of claims 2 through 8, wherein
- the part of the ring (6) situated inside the casing (5) comprises two fork-shaped holder parts (47), that is to say each comprising a pair of arms (55) and a connection body (56) connecting these arms (55) to the ring (6), the arms (55) of each fork being configured to be able to partially enclose the syringe body (2); and
- said arms (55) are made of a material having a degree of elastic flexibility which is such that they can be elastically deformed in order to be able to receive between them, and tightly grip, syringe bodies (2) of different external diameters.

10. The device as claimed in claim 9, wherein each of the arms (55) of a holder part (47) comprises at least one boss (58) protruding radially inward.

11. The device as claimed in claim 9 or claim 10, wherein the connection body (56) of each holder part (47) has an elbowed shape, that is to say comprises a base part, connected to the rest of the ring (6) and arranged substantially parallel to the axis of this ring (6), and an intermediate part, connected to said arms (55) and arranged substantially perpendicular to the axis of the ring (6), said base parts being arranged on the same diameter as that in which the wing-shaped parts (45) project.

12. The device as claimed in one of claims 9 through 11, wherein at least one of said holder parts (47) has a flexible structure, particularly in the area of its connection body (56), in such a way as to make it easier to join the syringe body to the ring (6).

13. The device as claimed in one of claims 9 through 12, wherein said holder parts (47) are designed in such a way as to press the joining means (12) of the syringe body (2) flat against the ring (6) or a defined portion (48) thereof.

14. The device as claimed in one of claims 2 through 13, wherein the protective casing (5) has wedge means (30, 31) with which it is possible to laterally wedge a syringe body (2) placed in this casing (5), these wedge means (30, 31) being radially movable in such a way as to be able to wedge syringe bodies (2) of different external diameters.

15. The device as claimed in one of claims 9 through 14, wherein:
- the syringe body (2) has a proximal flange (12) for joining it to the ring (6);
- the part of the ring (6) situated inside the casing (5) comprises the aforementioned holder parts (47), and
- the ring (6) comprises two joining parts (48) of curved shape which are arranged in such a way as to substantially form an annulus; these joining parts (48) are connected to the ring (6) in the area of peripheral zones and are made of a material having a degree of elastic flexibility; the peripheral zones of these joining parts (48) are situated from one another at a distance greater than the external diameter of said flange (12), while the median zones of these joining parts (48), in the nondeformed state of these joining parts (48), are situated from one another at a distance smaller than the external diameter of the flange (12); these median zones have, on the side of the ring (6) via which the syringe body (2) is engaged through the joining parts (48), bevels (60) which are configured to permit radial clearance of these median zones at the moment of this engagement.

16. The device as claimed in claim 15, wherein the ring (6) comprises two arc-shaped openings (35) which delimit said joining parts (48) and receive the casing (5) in them, these openings (35) having wedge shoulders (40) at their longitudinal ends which allow the ring (6) to be wedged in terms of rotation relative to the casing (5).

17. The device as claimed in one of claims 2 through 16, wherein the distal part (15) of the plunger rod (4) is continued, at the proximal end, beyond said bridge or bridges (20), and wherein this continuation (21) has an external diameter slightly smaller than the internal diameter of the proximal part (16) of this plunger rod (4).

18. The device for medical use, with safety, comprising
(a) a subassembly of the syringe type, as claimed in one of claims 1 through 17, comprising the elongate body (2) on a reference axis; the needle (11) arranged axially, communicating with the inside of said body, at the distal end thereof; the plunger (3) displaceable inside said body as far as a distal abutment position; and the plunger rod (4) emerging at the proximal end of said body (2), with the proximal support part (17);
(b) a safety subassembly, comprising the protective casing (5) mounted about the body (2), axially displaceable relative to the latter between two positions, namely an exposure position in which the needle (11) is exposed, and a safety position in which the needle (11) is entirely contained inside said protective casing (5);
in which device, in combination:
(i) the protective casing (5) is continued by a proximal portion (26) beyond the proximal end of the body (2) in the position of exposure of said protective casing, the plunger rod (4) emerging from the proximal end of said proximal portion (26);
(ii) the actuating ring (6) extends radially from the proximal end of the body (2), being integral with the latter, and includes an outer support part (45) either side of the body (2);
(iii) means (41) of axial sliding between the actuating ring (6) and the proximal portion (26) of the protective casing (5), said proximal portion being mounted freely in translation through the ring (6);
(iv) means (20) for axially retracting the plunger rod (4) on itself by means of an axial push on the proximal support part (17) when the plunger (3) is in its distal abutment position, so that said proximal support part (17) comes into abutment against the proximal end of the protective casing (5) then displaces the latter toward its safety position, again by pushing.
